Europäisches Patentamt

European Patent Office

Office européen des brevets

(11) Publication number: **0 283 752**
A2

# EUROPEAN PATENT APPLICATION

(21) Application number: 88102766.8

(22) Date of filing: 24.02.88

(51) Int. Cl.⁴: **G06F 3/023** , G06F 15/02

(30) Priority: 24.03.87 US 29848

(43) Date of publication of application:
28.09.88 Bulletin 88/39

(84) Designated Contracting States:
DE FR GB NL

(71) Applicant: **Hewlett-Packard Company**
**3000 Hanover Street**
**Palo Alto California 94304(US)**

(72) Inventor: **Fuchs, Kenneth J.**
**126 Woodridge Road**
**Wayland, MA 01778(US)**

(74) Representative: **Liesegang, Roland, Dr.-Ing. et al**
**FORRESTER & BOEHMERT**
**Widenmayerstrasse 4 Postfach 22 01 37**
**D-8000 München 22(DE)**

(54) **Portable terminal.**

(57) A portable terminal has a body (B) with a top and a bottom, a viewing screen (5) mounted at the top of said body that is viewable from one side thereof and forms alpha-numeric characters having their bottom facing the bottom of the body, a keyboard (K) mounted on said body (B) below said screen (5) and having alpha-numeric characters imprinted on the keys thereof with their bottoms facing the bottom of said body, and a rigid handle (H) joined to the bottom of said body so as to from a space (0) therebetween.

FIG 1

## PORTABLE TERMINAL

Vital statistics of a patient have been noted by hand on a chart attached to a patient's bed, but charts are gradually being replaced with portable terminals that can be placed in radio contact with a main computer where the information is stored. After the patient's name and the nurse's name have been typed on the keyboard of the terminal, the appropriate information can be brought up on the screen if the nurse is authorized to attend that particular patient. In prior terminals the arrangement of the screen, the keyboard, and the handle by which the terminal is carried, if there is one, is such that it is easier for the nurse to place the terminal on a surface when she wishes to operate it rather than hold it against her body with one hand and operate the requisite keys with the other. The reason for this is that the keyboard is too close to the body for easy reading and operation.

In accordance with this invention, the keyboard and the screen are mounted on the body of the terminal so that both can be read from the same direction, and the top of the keyboard is parallel to and linearly overlaps the bottom of the screen. A handle for carrying the terminal forms an opening for the fingers that is adjacent to the bottom of the keyboard and visible from the direction in which the screen and keyboard can be read. The surface of the handle remote from the keyboard is curved toward the keyboard.

Thus, when the nurse grasps the terminal at the end adjacent the top of the screen and presses the curved surface of the handle against her body, the screen and keyboard are firmly positioned away from her body so that the screen is easily seen and the keyboard is readily accessible for operation in spite of protuberances from the body.

Brief Description of the Drawings

Figure 1 is a plan view of the terminal of this invention taken from the reading direction,
Figure 2 is an elevation of Figure 1, and
Figure 3 is an end view of Figure 1.

Detailed Description of the Invention

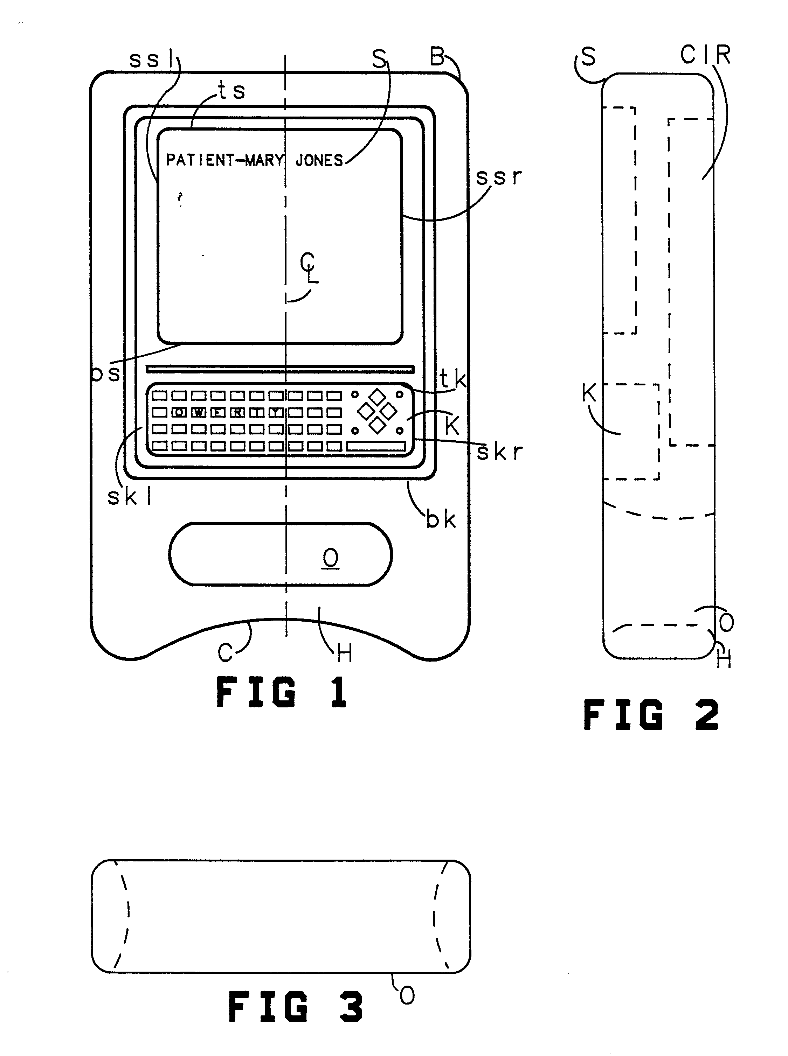

Reference is made to the plan view of a terminal of Figure 1 in which a viewing screen S having a top ts, a bottom bs and sides ssl and ssr is mounted on at the top of a body B of the terminal so as to be readable from one side of the body. A keyboard K having a top tk, a bottom bk and sides skl and skr is mounted on the body B below the screen S so that it is readable from the same side of the body B. In this particular illustration the centers of the screen S and the keyboard K are aligned on a vertical line $\mathcal{L}$, but one could be shifted horizontally with respect to the other. In any event the bottom bs of the screen S and the top tk of the keyboard K are longitudinally overlapped. A rigid handle H is joined to the bottom of the body B of the terminal and forms a space 0 between them through which the fingers can be pressed. The surface C of the handle that is remote from the keyboard K is shaped so as to curve toward the keyboard. The screen S, keyboard K and opening 0 are all visible from the same side of the body B, and the bottoms of alpha-numeric figures appearing on the screen and imprinted on the keys of the keyboard K face the bottom of the body B. When the terminal is oriented with the handle H at the bottom, the keyboard K is above it and the screen S is above the keyboard.

In the elevation view of Figure 2 the screen S and keyboard K are shown in the same plane, but they could be in different planes as long as both are readable from a common direction. The circuitry required for communicating with the central computer under the control of the keyboard K and forming characters on the screen is shown at CIR.

A significant advantage of the invention is that when the top of the body B adjacent to the top ts of the screen S is grasped in one hand and the curved surface C is pressed against the body, the handle H displaces the keyboard K away from any projections on the body so that it can be easily viewed.

### Claims

1. Portable terminal characterized by a body (B) having a top and bottom; a screen (S) mounted at the top of said body and viewable from one side thereof, said screen having means for forming alpha numeric characters therein having their bottoms facing the bottom of said body (B): a keyboard (K) mounted on said body below said screen (S) and viewable from the same side of said body (B) as said screen, said keyboard having keys on which alpha numeric characters are imprinted with their bottoms facing the bottom of said body; and a rigid handle (H) joined to the bottom of said body so as to provide a space (O) therebetween that is visible from said one side of body.

2. Portable terminal as set forth in claim 1, characterized in that a surface (C) of said rigid handle (H) that is remote from said keyboard (K) is curved toward said keyboard.

FIG 1

FIG 2

FIG 3